Europäisches Patentamt

European Patent Office

Office européen des brevets

(11) Publication number: **0 414 370 A1**

# EUROPEAN PATENT APPLICATION

(21) Application number: 90307740.2

(22) Date of filing: 16.07.90

(51) Int. Cl.⁵: **C07C 17/20, C07C 19/08**

(30) Priority: 24.07.89 US 383632
25.10.89 US 432368

(43) Date of publication of application:
**27.02.91 Bulletin 91/09**

(84) Designated Contracting States:
**AT BE CH DE ES FR GB GR IT LI LU NL SE**

(71) Applicant: **E.I. DU PONT DE NEMOURS AND COMPANY**
**1007 Market Street**
**Wilmington Delaware 19898(US)**

(72) Inventor: **Gumprecht, William Henry**
**2606 Stephenson Drive**
**Wilmington, Delaware 19808(US)**
Inventor: **Rimmer, Robert W.**

**deceased(US)**

(74) Representative: **Woodcraft, David Charles et al**
**BROOKES & MARTIN High Holborn House**
**52/54 High Holborn**
**London, WC1V 6SE(GB)**

(54) Halogen-exchange process.

(57) A halogen exchange process is disclosed for the preparation of fluorinated derivatives of pentachloroethane without substantial production of perhalogenated by-products thereof. In particular, the invention relates to such process which involves the use of high-fluoride content antimony pentahalides for the preparation of $CF_3CHCl_2$ - (HCFC-123), $CF_3CHClF$ (HCFC-124) and $CF_3CHF_2$ (HFC-125).

EP 0 414 370 A1

## HALOGEN-EXCHANGE PROCESS

BACKGROUND OF THE INVENTION

This invention relates to a halogen-exchange process for the preparation of fluorinated derivatives of pentachloroethane without substantial production of perhalogenated by-products thereof. In particular, the invention relates to such process which involves the use of high-fluoride-content antimony pentahalides for the preparation of $CF_3CHCl_2$ (HCFC-123), $CF_3CHClF$ (HCFC-124) and $CF_3CHF_2$ (HFC-125).

Processes are known for the preparation of fluorinated derivatives of pentachloroethane by reaction of an antimony pentachlorofluoride with an underfluorinated precursor thereof. In general, these processes suffer in that substantial amounts of perhalogenated by-products accompany the desired hydrogen-containing materials, especially at elevated temperatures. Such processes also suffer the disadvantage of usually employing relatively high proportions of hydrogen fluoride in association with an antimony pentachloride or chlorofluoride. This is disadvantageous in that use of the highly volatile hydrogen fluoride necessitates the use of expensive high pressure equipment, and that the hydrogen fluoride-antimony pentahalide system is highy corrosive to ordinary materials of construction.

Antimony pentafluoride (SbF5) has also been employed as a halogen exchange agent. For example, Benning et al., U.S. 2,490,764 disclose its use for fluorinating compounds of the formula $ACF_2(CF_2)_nCF_2B$, where n is an integer of at least I, A is H or Cl and B is Cl or F, to produce compounds of the formula $ACF_2(CF_2)_nCF_3$, where n is at least 1 and A is H, Cl or F. The highly fluorinated starting materials are extremely inert and require high temperatures (175-350°C) for their conversion to more highly fluorinated derivatives. The process is limited to materials having 3 or more carbons inasmuch as the corresponding pentafluorochlorethane, $CF_3CClF_2$, is completely inert to SbF5 under the disclosed conditions.

On the other hand, the prior art recognizes that reactions of SbF5 with raw materials that are generally regarded as more easily fluorinated are often difficult to control and lead to unsatisfactory results. Pacini et al., U.S. 3,287,424 teach that SbF5 produces very vigorous reactions, sometimes resulting in carbon-carbon bond cleavage, also that dehydrohalogenation can take place in which double bonds are created. Further, SbF5 is not efficiently utilized in that one mole is required per F radical introduced. Similarly, Davis, U.S. 3,201,483 discloses that attempts to fluorinate normally easily fluorinated organic compounds with SbF5 usually result in highly exothermic reactions giving perfluorinated products.

Although, as Pacini et al. imply, it would be desirable to utilize the full fluorinating potential of SbF5, that is, well beyond one fluoride atom, it has been found herein that this is not always possible or advisable, and can lead to perhalogenated by-products when starting with hydrogen-containing raw materials.

Perhalogenated by-products not only constitute a waste of valuable starting material and loss of yield, but are often difficult and costly to separate from the desired hydrogen-containing materials by fractional distillation.

The hydrogen-containing fluorinated ethanes are of current interest as environmentally-safe replacements for existing perhalogenated products because of the growing concern over the possible role of the latter compounds in destroying stratospheric ozone. For example, $CF_3CHCl_2$ is an environmentally-acceptable alternate to $CCl_3F$ (CFC-11) as a blowing agent, solvent, tobacco puffing agent and refrigerant. It is also a useful raw material for the preparation $CF_3CHClF$ and $CF_3CHF_2$, which are also environmentally acceptable. $CF_3CHClF$ is useful as a blowing agent, sterilant, propellant, refrigerant and raw material for preparing $CF_3CH_2F$, a zero-ozone-depletion-potential replacement for $CCl_2F_2$ (CFC-12) as a refrigerant. $CF_3CHF_2$ is a zero-ozone-depletion-potential replacement for R-502, which is an azeotropic blend of $CHClF_2$ (HCFC-22) and $CF_3CClF_2$ (CFC-115). It is also useful as raw material for preparing tetrafluoroethylene.

It is an object of this invention to provide a liquid-phase process for the preparation of at least one of $CF_3CHCl_2$, $CF_3CHClF$ and $CF_3CHF_2$ in high yields with minimal loss to perhalogenated by-products. Another object is to provide such a high yield process for high purity product which does not require the use of HF as halogen-exchange agent and thereby avoids HF-induced reactor corrosion and the need for high cost, high pressure equipment.

SUMMARY OF THE INVENTION

What has been discovered is a process for preparing at least one polyfluorinated hydrogen-containing ethane having the formula $CF_3CHXY$, where each of X and Y is Cl or F, which process comprises

contacting at least one precursor of said ethane, said precursor having the formula $CCl_{3-x}F_xCHCl_2$, where $x$ = 0 to 3, or $CF_3CHClF$ with a halogen exchange composition consisting essentially of $SbF_5$-$yCl_y$, where $y$ = 0 to 1, at a reaction temperature in the range of about 5°C to about 200°C effective to form a reaction mixture that contains at least one $CF_3CHXY$ compound, X and Y being as above, and is substantially free of perhalogenated by-products, such that during the contacting step

    (a) when $CCl_3CHCl_2$ is substantially present, the reaction temperature is maintained below about 30°C until the $CCl_3CHCl_2$ is substantially converted to $CCl_2FCHCl_2$;

    (b) when $CCl_2FCHCl_2$ is substantially present, where $y$ in $SbF_5$-$yCl_y$ is 1 or less and the reaction temperature is maintained below about 60°C until the $CCl_2FCHCl_2$ is substantially converted to $CClF_2CHCl_2$;

    (c) $y$ in $Sb_5$-$yCl_y$ is 1 or less when $CClF_2CHCl_2$ or $CF_3CHCl_2$ is substantially present and 0.5 or less when $CF_3CHClF$ is substantially present; and

    (d) the number of moles, $n$, per mole of precursor of $SbF_5$-$yCl_y$, the value of $y$ and $p$, the number of precursor Cl radicals to be replaced, are such that the relationship $(5-y)-(p/n)$ is at least 3 throughout the contacting step and at least 4 when the reaction temperature exceeds about 150°C, and thereafter, separating at least one polyfluorinated hydrogen-containing ethane having the formula $CF_3CHXY$, where each of X and Y is Cl or F, from the reaction mixture.

Another embodiment of the invention process comprises a process for preparing $CF_3CHCl_2$, with and without substantial coproduction of $CF_3CHClF$, wherein one mole of $CClF_2CHCl_2$ is contacted with at least about 0.5, preferably at least about 0.7 mole, of $SbF_5$-$yCl_y$, where $y$ is 1 or less and the ratio $(5-y)-(p/n)$ is at least about 3, preferably at least about 3.25 throughout the contacting step at an effective temperature of about 60°C to about 150°C.

Still another embodiment comprises a process as above for preparing $CF_3CHClF$, with and without coproduction of $CF_3CHF_2$, wherein $CF_3CHCl_2$ is reacted with at least about 0.7, preferably at least about 1 mole of $SbF_5$-$yCl_y$, where $y$ is 1 or less, preferably about 0.5 or less, and the ratio $(5-y)-(p/n)$ is at least about 3.5, preferably at least about 4, at an effective temperature of 60°C to about 200°C.

Still another embodiment comprises a process for preparing $CF_3CHF_2$ which comprises reacting at least one of $CF_3CHCl_2$ and $CF_3CHClF$ with at least about a molar proportion of $SbF_5$-$yCl_y$, where $y$ is 0.5 or less, preferably zero, at an effective temperature of from about 150°C to about 200°C, the number of moles, $n$, of said $SbF_5$-$yCl_y$ per mole of the chlorofluoroethane being such that the relationship $(5-y)-(p/n)$, where $p$ is 1 for $CF_3CHClF$ and 2 for $CF_3CHCl_2$, is equal to at least about 4.

## DETAILED DESCRIPTION OF THE INVENTION

Overall, the invention process provides $CF_3CHCl_2$, $CF_3CHClF$ and $CF_3CHF_2$ products in high yields at high conversions of the underfluorinated precursors thereof and substantially uncontaminated by perhalogenated by-products. That the precursors can be smoothly, cleanly and substantially completely converted to the desired tri-, tetra- and pentafluorinated materials is surprisingly in view of the difficulties encountered in other antimony pentahalide-based halogen exchange processes and the well-documented difficulty of fluorinating -CHCl_2 and -CHClF groups when adjacent to the strongly deactivating -CF_3 group. By substantially free of perhalogenated by-products is meant that the content of such impurities in the product mixture is less than about 5 mole %, preferably less than about 3 mole % and more preferably less than about 1 mole %.

By substantially converted is meant at least about 95% of the indicated organic starting material is converted to the next higher fluorine-content material.

By substantially present is meant that the indicated organic starting material is present as a major amount of the material to be fluorinated.

The invention involves contacting $CCl_3CHCl_2$, $CCl_2FCHCl_2$, $CClF_2CHCl_2$ and/or $CF_3CHCl_2$ with an Sb pentahalide as defined. If desired, $CF_3CHClF$ can also be used as starting material in the production of $CF_3CHF_2$. Contact between the starting material and the Sb pentahalide can be achieved though agitation, shaking or otherwise mixing the halocarbon reactant into the Sb pentahalide phase using any of the techniques of the art.

In the reaction that occurs, one or more Cl radicals of the starting material are replaced by F radicals of the Sb pentahalide as illustrated in the following equations with $SbF_5$ as halogen exchange agent:

$$CClF_2CHCl_2 + SbF_5 \longrightarrow CF_3CHCl_2 + SbF_4Cl \quad (1)$$
$$CF_3CHCl_2 + SbF_5 \longrightarrow CF_3CHClF + SbF_4Cl \quad (2)$$
$$CF_3CHClF + SbF_5 \longrightarrow CF_3CHF_2 + SbF_4Cl \quad (3)$$

In some instances, as when the starting material is F radical-free or contains fewer than four F radicals, $SbF_4Cl$ may also participate in the process, as illustrated in the following equation:

$$CCl_3CHCl_2 + SbF_4Cl \longrightarrow CCl_2FCHCl_2 + SbF_3Cl_2 \qquad (4)$$

It is preferred, however, to avoid or minimize involvement of $SbF_4Cl$ as halogen exchange agent for the present purpose at temperatures above about $60°C$ since it can lead to the formation of $SbF_3Cl_2$, as per equation (5).

$$CCl_2FCHCl_2 + SbF_4Cl \longrightarrow CClF_2CHCl_2 + SbF_3Cl_2 \qquad (5)$$

This is undesirable since $SbF_3Cl_2$ has been found to be an active chlorinating agent at temperatures above about $60°C$, attacking C-H bonds, so that its presence in the reaction mixture in undue proportions may lead to excessive amounts of perhalogenated by-products.

The $SbF_{5-y}Cl_y$ reactant includes the well-known compositions $SbF_5$ and $SbF_4Cl$, defined by $y = 0$ and 1, and mixtures thereof. It also includes related compositions defined by fractional values of $y$ between 0 and 1 for which no simple structural formula can be drawn, but which can likewise be prepared by known processes, including the reaction of HF with $SbCl_5$, or reaction of $SbF_5$ with $SbCl_5$, or reaction of $SbF_3$ with $F_2$ or ClF, in the stoichiometrically required proportions. Preferred are the low-chloride-content compositions defined by $y =$ less than 1.0, more preferably 0.5 or less, and most preferably zero, corresponding to $SbF_5$. Low chloride Sb pentahalides are preferred because of their greater halogen exchange activities, and the superior results they provide.

The quantity of the Sb pentahalide employed may range widely and will depend on the particular organic precursor employed and its ease of fluorination via halogen exchange, the operating temperature and the result desired.

Sufficient amounts of the Sb pentahalide will ordinarily be used, however, to provide at least one F radical for every Cl radical to be replaced. As stated earlier, it is a feature of the invention to control the reaction parameters so as to minimize the formation of $SbF_3Cl_2$ via $SbF_4Cl$ and, in this way, minimize the production of perhalo by-products. The relationship $(5-y)-(p/n)$, where $n$ is the number of moles per mole of organic precursor of the $SbF_{5-y}Cl_y$, $5-y$ is its number of F radicals, and $p$ is the number of Cl radicals of the precursor to be replaced, i.e., the number of halogen exchange stages to be effected, defines the number of F radicals expected in the residual Sb compound at the conclusion of the reaction.

The greater the number of molar proportions of the Sb pentahalide, the smaller the value of $y$ and the lower the temperature, the greater will be the fluoride content of the residual Sb pentahalide, and the smaller will be the amount of perhalo by-products produced. Thus, it is desirable to adjust the composition of the Sb pentahalide and its molar proportion such that the value of $(5-y)-(p/n)$ will be at least 3 at the end of each halogen exchange stage of the reaction, preferably at least 3.25, more preferably at least 3.5 and most preferably at least 4.0, depending on the organic reactant and the reaction temperature. Additional $SbF_5$ or other high fluoride-content Sb pentachlorofluorides can be added to the reaction mixture, as needed, as the reaction proceeds, for example, as when the temperature is raised stepwise during the course of an overall fluorination reaction from $CCl_3CHCl_2$ to $CF_3CHF_2$, to maintain the value of $(5-y)-(p/n)$ greater than or equal to 3 throughout each halogen exchange stage. In other words, the fluoride content of the residual antimony pentachlorofluoride throughout the reaction will correspond to at least that of $SbF_3Cl_2$ or at least 22.8% by weight of fluoride.

The number of halogen exchange stages, $p$, i.e., the number of precursor Cl radicals to be replaced by F radicals, can vary from 1 to 5 depending on the precursor and the desired fluorinated product. For example, the conversion of $CCl_3CHCl_2$ to $CCl_2FCHCl_2$ involves one halogen exchange stage; its conversion to $CF_3CHF_2$, five stages. The number of F radicals in the residual antimony pentahalide after $p$ halogen exchange stages is given by the expression $(5-y)-(p/n)$, where $5-y$ is the number of F radicals in the starting Sb pentahalide, $n$ is the number of moles of Sb pentahalide employed per mole of precursor, and $p$ is the number of halogen exchange stages. When a single halogen exchange stage is effected, the number of F radicals in the residual Sb pentahalide is equal to $(5-y)-(1/n)$. When more than one halogen exchange stage is effected, e.g., 3 stages, the number of F radicals in the residual Sb pentahalide is given by $(5-y)-(p/n)$, where $p$ in this instance is 3. In accordance with the invention, $(5-y)$ and $n$ of the starting Sb pentahalide will be chosen such that $(5-y)-(p/n)$ will always be equal to or greater than 3 throughout the contacting step and at the end of the desired $p$ number of stages to be effected. Stated another way, the minimum number of moles, $n$, of $SbF_{5-y}Cl_y$ per mole of precursor needed to result in a residual Sb pentachlorofluoride having 3 or more F radicals, $z$, after $p$ halogen exchange stages, is given by the expression: $n$ is greater than or equal to $p/[(5-y) - (z)]$.

It will be understood that $p$ can be a fractional number as when a mixture of halogen exchange products is formed. Thus, in accompanying Example 1 involving 0.75 mole $SbF_5$ per mole $CClF_2CHCl_2$ there is produced about 7% $CF_3CHClF$ along with about 92% of $CF_3CHCl_2$ which corresponds to about 1.07 halogen exchange stages. The fluoride content of the residual Sb pentachlorofluoride is calculated as (5-y)-(1.07/0.75) about 3.6, which is well above the requirement that this relationship be equal to or greater than 3. In Example 2, involving 1.46 moles $SbF_5$ per mole $CClF_2CHCl_2$, there is produced about 58% $CF_3CHClF$ along with about 40% $CF_3CHCl_2$. The fluoride content of the residual Sb pentachlorofluoride is calculated to be (5-y)-(1.58/1.46) = about 3.9, which is well above the at least 3 requirement.

Excess $SbF_5$, which is normally liquid, also provides for a fluid reaction mass, thereby promoting the halogen exchange reaction through good mixing of the reactants under agitation, resulting in higher conversions of the organic component and fewer side-products. Fluid, more-easily-agitated reaction mixtures may also be provided by employing a liquid diluent inert to the reactants under the reaction conditions, such as perfluorocyclohexane, $H(CF_2)_6F$, $F(CF_2)_7F$ and the like highly fluorinated alkanes disclosed in Benning U.S. 2,490,764.

It will be noted that HF is not needed as halogen exchange agent. However, if HF is present it will preferably be held to proportions less than one mole per mole of the Sb pentahalide, most preferably will be substantially absent, since its presence increases the corrosivity of the reaction mass to ordinary materials of reactor system construction.

Reaction temperatures can vary from near $0°C$ to about $200°C$ depending on the starting material; the lower the fluorine content of the organic starting material, the lower should be the temperature to avoid uncontrolled reactions that lead eventually to perhalogenated by-products. Fluorination of $CCl_3CHCl_2$ to $CCl_2FCHCl_2$ is best conducted below $30°C$, that of $CCl_2FCHCl_2$ to $CClF_2CHCl_2$ between about $30°C$ and $60°C$, and that of $CClF_2CHCl_2$ to $CF_3CHCl_2$ between $60°C$ and $120°C$, preferably between $80°C$ and $120°C$. Temperatures for fluorinating $CF_3CHCl_2$ to $CF_3CHClF$ overlap those for the production of $CF_3CHCl_2$ and can be as high as $150°C$, with about $70°C$ to about $120°C$ preferred. Both conversions are conveniently carried out in a single step. Fluorination of $CF_3CHClF$ to $CF_3CHF_2$ requires higher temperatures, normally between about $150°C$ and $200°C$.

At temperatures above about $100°C$ it is advisable to maintain as high a fluoride content in the pentavalent antimony compound as is practicable---preferably approaching $SbF_5$---and at a high molar proportion relative to that of the organic precursor so that the relationship of the fluoride content of the antimony compound to its concentration in the reaction, as defined by (5-y)-(1/n) for each halogen exchange stage, is at or close to 4 to minimize production of perhalo compounds at the expense of the desired product(s).

Pressure is not critical. Atmospheric and autogenous pressures are most convenient and are therefore preferred.

The process may be conducted batchwise in a closed or ventable system. It may also be conducted in a continuous manner with the product(s) taken off intermittently or continuously. In addition, the process may be operated in a semi-continuous manner by reacting the organic precursor with a high-fluoride-content Sb pentachlorofluoride until the fluorinating power of the Sb pentachlorofluoride is reduced and thereafter treating the reduced fluoride-content Sb pentachlorofluoride with $SbF_5$ or with HF to regenerate a high- fluoride-content Sb pentachlorofluoride by methods known in the art, and recycling it to the process reactor. Since $CF_3CHCl_2$, $CF_3CHClF$ and $CF_3CHF_2$ are all gaseous at temperatures above $30°C$ at atmospheric pressure, it is convenient to bleed off a portion of the vapor phase intermittently or continuously through a pressure-control valve. This may be done while feeding the appropriate underfluorinated organic precursor to the reactor.

The volatile reaction product mixture exiting the reactor contains the hydrogen-containing product(s), unreacted starting material, if any, and by-products, if any. It can be resolved by any of a variety of well-known techniques, including washing with aqueous caustic, then water and drying. A preferred isolation procedure involves scrubbing in aqueous HCl precooled to about $-50°$ or $-60°C$. This facilitates collection of all products as liquids. The scrubbed products can be further purified by fractional distillation. Unreacted or incompletely fluorinated material can be recycled to the reactor.

The reaction vessel is constructed from materials which are resistant to the action of the reactants. Examples include stainless steels, high nickel alloys such as "Hastelloy" and "Inconel", and plastics such as polyethylene, polypropylene, polychlorotrifluoroethylene and polytetrafluoroethylene.

Examples

In the following illustrative examples the product mixtures were analyzed by gas chromatography and mass spectroscopy. Analyses are in area percent.

Example 1

A 95.8 gram portion of SbF$_5$ (0.442 mole) was poured into a 150 ml. stainless steel cylinder equipped with a polytetrafluoroethylene-coated, magnetically-actuatable stirring bar and contained in an argon-filled dry box. An appropriate valve was attached. After pressure-testing the cylinder to 500 psig with dry N$_2$ gas, it was cooled in a solid CO$_2$-methanol mixture, vented and evacuated. A 100 gram portion of CClF$_2$CHCl$_2$ - (0.590 mole, 99.98% gas-chromatographically pure) was then sucked into the cooled and evacuated cylinder from an inverted reservoir cylinder. The reactor cylinder was then placed in an oil bath preheated to 100° C, and the mixture was agitated for 3 hours. Volatiles from the reaction vessel were vented into 20.7% aqueous hydrochloric acid, precooled to -50° C, while heating the reactor to 120° C. The organic layer that formed (81.1 grams) was collected at below 0° C in a vessel containing a mixture of anhydrous Na$_2$CO$_3$ and Na$_2$SO$_4$ to absorb and remove any residual acid and water. It was sampled at room temperature into an evacuated gas bulb and analyzed gas chromatographically and mass spectroscopically. The results are presented in the accompanying Table.

The nonvolatile residue left in the reactor weighed 109 grams; on this basis, assuming it was free of organic material, it corresponded to a mixture of SbF$_3$Cl$_2$ (86%) and SbF$_4$Cl (14%), i.e., SbF$_{3.14}$Cl$_{1.86}$. The volatile and non-volatile fractions together amounted to 190 grams or 97.0% of the materials charged to the reactor.

Example 2

The procedure of Example 1 was repeated except that 189.9 grams (0.876 mole) of SbF$_5$ and 101.6 grams (0.60 mole) of CClF$_2$CHI$_2$ were employed. The volatilized portion of the reaction mixture weighed 69.3 grams, and non-volatile residue in the reactor 209 grams. Assuming the latter was free of organic material, it corresponded to a residual Sb pentachlorofluoride having the empirical formula of SbF$_{3.67}$Cl$_{1.33}$. The combined weight, 278 grams, corresponded to 95.6% of the materials employed.

The analytic results for the organic product are given in the accompanying Table.

Table

|  | Ex. 1 | Ex. 2 |
|---|---|---|
| CClF$_2$CHCl$_2$ (unreacted) | 0.12 | 0.37 |
| CF$_3$CHCl$_2$ | 91.98 | 40.42 |
| CClF$_2$CHClF | 0.03 | -- |
| CF$_3$CHClF | 7.42 | 58.32 |
| CClF$_2$CHF$_2$ | 0.06 | 0.02 |
| CF$_3$CHF$_2$ | 0.01 | 0.21 |
| CClF$_2$CCl$_2$F | 0.19 | -- |
| CClF$_2$CClF$_2$ | 0.20 | 0.53 |
| CF$_3$CClF$_2$ | less than 0.01 | 0.04 |
| Other (unidentified) | -- | 0.09 |
|  | 100.02 | 100.00 |

The data in the Table show that the starting material, CClF$_2$CHCl$_2$ is practically completely converted to the desired hydrogen containing products, CF$_3$CHCl$_2$ and CF$_3$CHClF, and also to isomers thereof in small amounts.

The data also show that perhalo by-products, namely CClF$_2$CCl$_2$F (CFC-113), CClF$_2$CClF$_2$ (CFC-114), and CF$_3$CClF$_2$ (CFC-115) are substantially absent, amounting to only 0.40% in Example 1 and 0.57% in Example 2.

The data also indicate that the invention process is amenable to control such that either one or both of the desired products, CF$_3$CHCl$_2$ and CF$_3$CHClF, can be produced as the predominating product in the reaction mixture.

6

Example 3

This example illustrates another preparation of $CF_3CHClF$. The reactor consisted of a 5-gallon, 316 stainless steel autoclave, electrically heated and equipped with a dip-leg, heating coil, magnetically-actuated agitator and a two-foot riser packed with 0.5 inch stainless steel helices. The reactor was evacuated, and $SbF_5$ (25.7 lbs = 0.12 lb-mole) was sucked into the reactor under a blanket of dry $N_2$ gas at ambient temperature. Then the $CF_3CHCl_2$ (23.1 lbs = 0.15 lb-mole, b.p. 29°C), precooled to 0-5°C to minimize vapor loss, was charged to the evacuated reactor. The reaction mixture was heated with stirring to 80°C and was maintained at 80°C for about 3 hours. During this period a maximum pressure of about 170 psig was reached in about 2 hours. The temperature was then lowered to 50-60°C, and the volatile products were vented off at a rate of 1-1.25 lbs/hour through a trap, water and caustic scrubbers, and 4AXH6 molecular sieve drier into a solid $CO_2$-cooled stainless steel cylinder. Toward the end of the venting cycle the temperature was increased to 85-90°C to ensure substantially complete removal of the organics and to maintain the residual antimony pentachlorofluoride, estimated to consist essentially of about $SbF_{3.7}Cl_{1.3}$, molten to facilitate recovery from the reactor.

The yield of the crude product mixture was 20 lbs, from which 15.2 lbs of $CF_3CHClF$ (99.98% pure, 92% based on $SbF_5$) was obtained by fractional distillation. The foreshot and heel, rich in $CF_3CHClF$ and essentially free of perhalogenated products, were saved for consolidation with subsequently produced lots of crude $CF_3CHClF$ to reduce losses.

Under the above conditions the yield of $CF_3CHF_2$ was about 0.1%. Repeating the process at 120°C did not result in an increase in the production of $CF_3CHF_2$. Repeating the process at 170°C resulted in the conversion of $CF_3CHCl_2$ to both $CF_3CHClF$ and $CF_3CHF_2$ without co-production of perhalogenated by-products. In marked contrast, $SbF_4Cl$, in place of $SbF_5$, at 170°C converted $CF_3CHCl_2$ only slowly to $CF_3CHClF$. This reaction, moreover, was accompanied by side reations resulting in the formation of about 10% of the perhalo by-products $CClF_2CClF_2$ and $CF_3CClF_2$.

Example 4

This example illustrates the preparation of $CF_3CHF_2$. $CF_3CHClF$ was reacted with an excess of $SbF_5$ at 150°C. The $CF_3CHF_2$ formed slowly. Formation of perhalogenated material was not observed. Conducting the reaction at 170°C resulted in enhanced rate of production of the desired $CF_3CHF_2$ material.

**Claims**

1. A process for preparing at least one polyfluorinated hydrogen-containing ethane having the formula $CF_3CHXY$, where each of x and Y is Cl or F, which process comprises
contacting at least one precursor of said ethane, said precursor having the formula $CCl_{3-x}F_xCHCl_2$, where x = 0 to 3, or $CF_3CHClF$ with a halogen exchange composition consisting essentially of $SbF_{5-y}Cl_y$, where y = 0 to 1, at a reaction temperature in the range of about 5°C to about 200°C effective to form a reaction mixture that contains at least one $CF_3CHXY$ compound, X and Y being as above, and is substantially free of perhalogenated by-products, such that during the contacting step
(a) when $CCl_3CHCl_2$ is substantially present, the reaction temperature is maintained below about 30°C until the $CCl_3CHCl_2$ is substantially converted to $CCl_2FCHCl_2$;
(b) when $CCl_2FCHCl_2$ is substantially present, the reaction temperature is maintained below about 60°C until the $CCl_2FCHCl_2$ is substantially converted to $CClF_2CHCl_2$;
(c) y in $Sb_{5-y}Cl_y$ is 1 or less when $CClF_2CHCl_2$ or $CF_3CHCl_2$ is substantially present and 0.5 or less when $CF_3CHClF$ is substantially present; and
(d) the number of moles, n, per mole of precursor of $SbF_{5-y}Cl_y$, the value of y and of p, the number of precursor Cl radicals to be replaced, are such that the relationship $(5-y)-(p/n)$ is at least 3 throughout the contacting step and at least 4 when the reaction temperature exceeds about 150°C, and thereafter,
separating at least one polyfluorinated hydrogen-containing ethane having the formula $CF_3CHXY$, where each of X and Y is Cl or F, from the reaction mixture.

2. A process of preparing $CF_3CHCl_2$ comprising
contacting $CClF_2CHCl_2$ with at least about 0.5 mole per mole of $CClF_2CHCl_2$ of $SbF_{5-y}Cl_y$, where y = 1 or less, and the relationship $(5-y)-(1/n)$, where n, the number of moles of $SbF_{5-y}Cl_y$ per mole of $CClF_2CHCl_2$, is at least about 3 throughout the contacting step at an effective reaction temperature of from about 60°C to about 120° C to form a reaction mixture that contains $CF_3CHCl_2$ and is substantially free of perhalogenated by-products, and thereafter

separating $CF_3CHCl_2$ from the reaction mixture.

3. The process of Claim 2 wherein the reaction temperature is from about 80° C to about 120° C, and n is in the range of from about 0.7 to about 5.

4. A process for preparing at least one of $CF_3CHCl_2$ and $CF_3CHClF$ comprising

contacting $CClF_2CHCl_2$ with at least about 0.5 mole permole of $CClF_2CHCl_2$ of $SbF_{5-y}Cl_y$, where y = 1 or less, and the relationship (5-y)-(p/n), where n, the number of moles of $SbF_{5-y}Cl_y$ per mole of $CClF_2CHCl_2$, is at least about 3 throughout the contacting step and p is about 1 for the production of $CF_3CHCl_2$ and is about 2 for the production of $CF_3CHClF$, at an effective reaction temperature of about 60° C to about 150° C to form a reaction mixture that contains at least one of $CF_3CHCl_2$ and $CF_3CHClF$ and is substantially free of perhalogenated by-products, and thereafter

separating at least one of $CF_3CHCl_2$ and $CF_3CHClF$ from the reaction mixture.

5. A process for preparing $CF_3CHClF$ comprising

contacting $CF_3CHCl_2$ with at least about 0.5 mole per mole of $CF_3CHCl_2$ of $SbF_{5-y}Cl_y$, where y is less than 1 and the relationship (5-y)-(1/n), where n, the number of moles of $SbF_{5-y}Cl_y$ per mole of $CF_3CHCl_2$, is at least about 3.5 at an effective reaction temperature of about 70° C to about 150° C to form a reaction mixture that contains $CF_3CHClF$ and is substantially free of perhalogenated by-products, and thereafter separating $CF_3CHClF$ from the reaction mixture.

6. The process of preparing $CF_3CHF_2$ comprising contacting $CF_3CHClF$ with at least about a one molar proportion of $SbF_{5-y}Cl_y$, where y is about 0.5 or less, and the molar proportion of said $SbF_{5-y}Cl_y$, n, is such that the relationship (5-y) - (1/n) is equal to at least about 4 at an effective reaction temperature of about 150° C to about 200° C to form a reaction mixture that contains $CF_3CHF_2$ and is substantially free of perhalogenated by-products, and thereafter

separating $CF_3CHF_2$ from the reaction mixture.

European
Patent Office

**EUROPEAN SEARCH
REPORT**

Application Number

**EP 90 30 7740**

## DOCUMENTS CONSIDERED TO BE RELEVANT

| Category | Citation of document with indication, where appropriate, of relevant passages | Relevant to claim | — CLASSIFICATION OF THE APPLICATION (Int. Cl.5) |
|---|---|---|---|
| A | EP-A-0 129 863 (DU PONT)<br>* claim 1 * | 1 | C 07 C 17/20<br>C 07 C 19/08 |
| A | EP-A-0 300 724 (ICI)<br>* claims 1,2 * | 1 | |
| A | GB-A-5 891 67 (ICI)<br>* the whole document * | 1 | |
| P,A | US-A-4 851 595 (W.H. GUMPRECHT)<br>* claims 1-3 * | 1 | |
| A | US-A-2 499 833 (M.A. PERKINS)<br>* column 2, line 45 - column 3, line 24 * | 1 | |
| A | US-A-2 549 988 (M.A. PERKINS)<br>* column 1, line 36 - column 2, line 32 * | 1 | |
| | | | TECHNICAL FIELDS SEARCHED (Int. Cl.5) |
| | | | C 07 C 17/00<br>C 07 C 19/00 |

The present search report has been drawn up for all claims

| Place of search | Date of completion of search | Examiner |
|---|---|---|
| Berlin | 26 October 90 | PROBERT C.L. |

CATEGORY OF CITED DOCUMENTS

X : particularly relevant if taken alone
Y : particularly relevant if combined with another
document of the same catagory
A : technological background
O : non-written disclosure
P : intermediate document
T : theory or principle underlying the invention

E : earlier patent document, but published on, or after
the filing date
D : document cited in the application
L : document cited for other reasons

&: member of the same patent family, corresponding
document